Europäisches Patentamt

European Patent Office

Office européen des brevets

(11) Publication number: **0 104 256**
**B1**

(19)

# EUROPEAN PATENT SPECIFICATION

(12)

(45) Date of publication of patent specification: 27.12.89

(21) Application number: 83902978.2

(22) Date of filing: 07.03.83

(86) International application number:
PCT/US83/00303

(87) International publication number:
WO 83/03364 13.10.83 Gazette 83/24

(51) Int. Cl.⁴: **B 01 D 27/06**, B 01 D 29/06,
A 61 M 5/16

(54) LOW VOLUME, LARGE AREA FILTERS FOR IV OR BLOOD FILTRATION.

(30) Priority: 30.03.82 US 363632

(43) Date of publication of application:
04.04.84 Bulletin 84/14

(45) Publication of the grant of the patent:
27.12.89 Bulletin 89/52

(84) Designated Contracting States:
BE DE FR GB

(56) References cited:
DE-A-2 523 013
DE-B-1 097 087
JP-A-53 113 371
US-A-3 200 953
US-A-3 486 626
US-A-3 486 626
US-A-3 746 595
US-A-3 954 623
US-A-4 013 072
US-A-4 046 696
US-A-4 130 622

(73) Proprietor: BAXTER INTERNATIONAL INC. (a
Delaware corporation)
One Baxter Parkway
Deerfield Illinois 60015 (US)

(72) Inventor: KERSTEN, Jean
259 Chaussee de Tournai
B-Villers St. Amand (BE)
Inventor: MATHIAS, Jean Marie
Rue Du Chene
1400 Nivelles (BE)

(74) Representative: MacGregor, Gordon et al
ERIC POTTER & CLARKSON 14 Oxford Street
Nottingham, NG1 5BP (GB)

Courier Press, Leamington Spa, England.

## Description

### Background of the invention

There is disclosed herein a filter element, and in particular, a large surface area filter element for use in medical applications.

In many medical applications fluids, such as blood, intravenous solutions, etc., are filtered to remove undesirable filtratable constituents. In some applications, such as blood filtration, surface-type or depth-type filter media is used. Surface-type media is usually a web of open-mesh material which is held, by a frame, in a filtering position. Depth-type media is usually a pad of fibrous or porous material.

In blood administration sets, blood or plasma is conducted from a reservoir, usually a flexible plastic bag, through a drip chamber and then to the patient via a catheter. Such sets are sold by Travenol Laboratories, Inc., Deerfield, Illinois, under product code numbers such as 2C2037 and 2C2157.

The drip chamber may include a filter element so that blood entering the chamber flows through the filter element and then exits the chamber. The drip chamber controls the delivery of fluid on a drop-by-drop basis.

In some constructions, the drip chamber includes a cylindrical housing, a cylindrical filter element that fits into the housing, and a cap that seals the chamber. The filter element includes an injection molded frame and a surface-type or depth-type filter media supported by the frame. The filter media has a generally cylindrical surface. The molding technique employs a split mold cavity and core which permits injection molding of the frame and attachment of the filter medium in one step. This is sometimes referred to as overmolding.

It has been determined that in some applications, it is desirable to increase the filtering ability of the filter. However, it may be undesirable to increase the length or width of the filter as that would require changing the geometry (i.e., size and shape) of the drip chamber. As is evident from US—A—3 954 623 and US—A—3 486 626, pleating of a filter can increase the surface area, but pleated filters have not been made using molding techniques as pleated filters include undercuts or re-entrant angles that would preclude opening of the mold to remove the filter element without destruction of the element. It is therefore an object of this invention to increase the filtering capacity of a medical filter element without changing the size and shape of the related housing. It is another object of this invention to increase the filtering capacity of a medical filter element while still permitting the filter element to be manufactured by molding.

According to the invention, there is provided a flow-through medical filter element which includes a filter medium and frame-like means for supporting and positioning said filter medium, wherein:

(a) said frame-like means is a molded member and includes a ring-like inlet end, a fluid-impervious terminal end spaced from said ring-like inlet, and a plurality of rib-like members which extend between and interconnect the inlet end and terminal end, said inlet end also having internally-extending pleat-supporting projections and said terminal and having complementary external pleat-supporting shoulders, said frame-like means being symmetrical about an axial plane and said external shoulders being formed at angles which are not re-entrant with respect to the said axial plane and which permit separation of a mold along the axial plane and release of the part; and

(b) said filter medium having longitudinal pleats therein defining angles which are not re-entrant with respect to the said axial plane and said medium being bonded to the internal pleat-supporting projections at the inlet end and to the external pleat-supporting shoulders at the terminal end.

By the phrase "said external shoulders being formed at angles which are not re-entrant, we mean that there would be no overlap in the surface lines of the shoulders when projected onto the said axial plane.

This construction and method of molding have been found to provide ease of fabrication and a high filtration surface area filter element.

### Brief description of the drawings

Figure 1 is an elevational view showing a partially broken away drip chamber with a filter element in place;

Figure 2 is a perspective view of a greatly enlarged pleated filter element;

Figure 3 is an elevational view of the bottom or terminal end of the filter element;

Figure 4 is an elevational view of the top or inlet end of the filter element;

Figure 5 is a greatly enlarged view of one section of the end plate showing the angular relationships for the pleat-forming surfaces;

Figure 6 is a diagrammatic view showing the mold cavities and core for forming a pleated filter element; and

Figures 7, 8 and 9 are perspective and elevational views of another filter element configuration.

### Description of the preferred embodiment

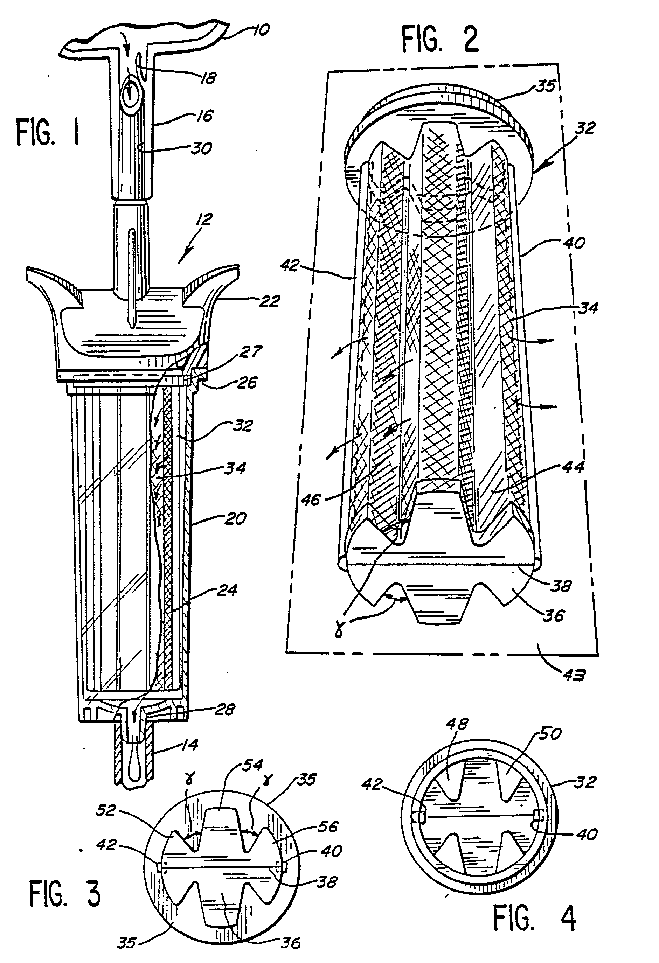

Referring now to the drawings, there is shown, in Figure 1, a blood plasma reservoir 10, a drip chamber assembly 12, and catheter tubing 14. The bag 10 includes an outlet tube 16 having a pierceable membrane seal 18.

The drip chamber 12 includes a cylindrical housing or body 20, a cap-and-spike assembly 22, and a filter element 24. The housing 20 has an open top 26 which has a stepped shoulder 27 for supporting the filter 24 and a drop outlet 28 at the bottom or terminal end. The cap 22 is sealed to the top 26 of the housing 20 and has a fluid conducting spike 30 for insertion into the outlet tube 16 and for puncturing the seal 18. The spike thus provides the inlet by which blood or other liquids can flow from the bag 10 to the filter 24. The filter element includes a support frame 32 and the filter media 34.

In operation, blood exiting the bag 10 flows into the chamber spike 30 and downwardly into the drip chamber body 20. The blood enters the top of the filter element 24 and then flows outwardly through the filter media and downwardly to the bottom of the body 20. The blood exits the housing 20 through the outlet 28 which is connected to the catheter tubing 14.

The filter element 24 is best seen in Figure 2. The frame 32 includes an upper ring-like inlet end 35, a flat, fluid-impervious terminal end plate 36 on which the parting line 38 can be seen and a pair of interconnecting side ribs 40 and 42. A parting line plane 43 passes through the element along the mold parting line and the longitudiinal axis of the body lies in the parting line plane.

The filter element is symmetrical with respect to the parting line plane. The surface filter media 34 is bonded to the frame and includes pleats such as 44 and 46. The pleats are formed during molding and are shaped in cooperation with internal projections such as 48 and 50 on the inlet end 35 and external groove forming shoulders such as 52, 54 and 56 on the terminal end 36.

The pleats are formed such that the pleat angle gamma (γ) opens outwardly from the parting line 38 so as to permit retraction of mold parts.

The pleat angle (γ) can be defined by its angular relations to the parting line. But it is more convenient to define the angles relative to a line 57 parallel to the parting line 38. The external shoulders 52, 54 and 56 define surfaces which in turn define grooves that are aligned with the pleats. For example, the shoulder 52 defines a first groove surface 53 and the shoulder 54 defines a second groove surface 55. The first surface 53 forms an acute angle alpha (α) (i.e., less than 90 degrees) with the parting line plane, or the parallel line 57, and the second surface 55 forms an obtuse angle (β) (i.e., greater than 90 degrees) with the parting line plane, or parallel line 57. It can be seen that these angles can be measured at line 57 or by projections to the parting line. The second groove defined by shoulders 54 and 56 exhibits similar but mirror image relationships.

Turning now to Figure 4, the surface of the inlet projections 48 and 50 also define the alpha (α), beta (β), and gamma (γ) angular relationships as the terminal plate grooves. The alignment of the shoulders and grooves cooperate in forming the pleats of the filter media.

When using surface-type filter media, the filter element 24 is molded by providing a mold core 60 which has been shaped to provide the desired internal shape. A split mold cavity 62 and 64 is then provided which can surround the core and provide molding spaces between the core and closed cavity. The filter media 34 is then applied and positioned on the core, the cavity closed, and the frame forming plastic injected. The plastic bonds to the media, thus forming an integral part, and the heat sets the filter media to form the pleats.

Thereafter, the cavity is opened, the core is retracted and the molded element is ejected. This molding can take place because of the shape and positioning of the pleat grooves.

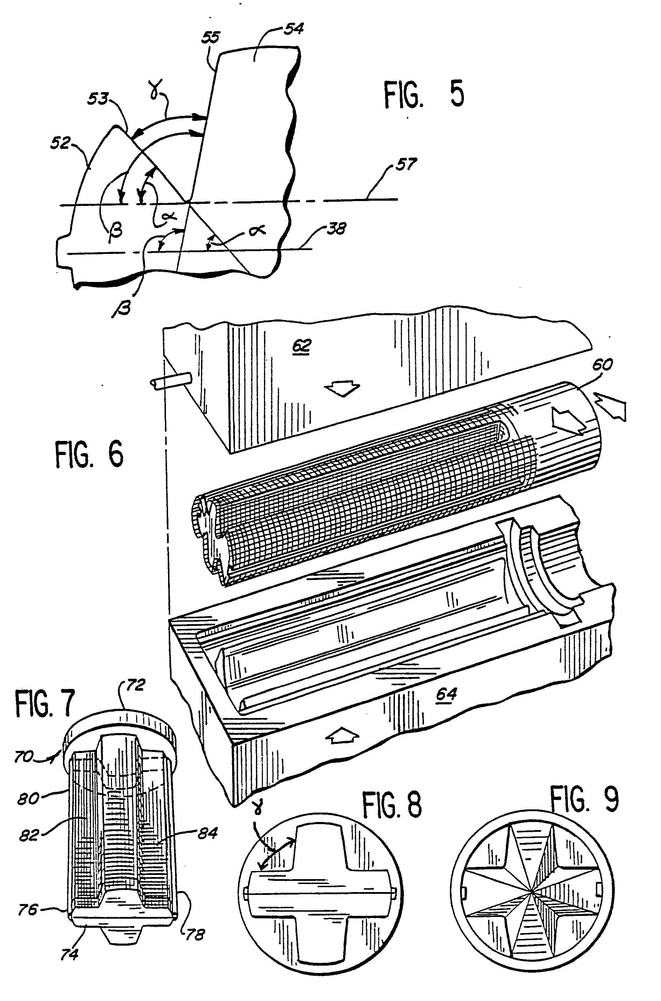

A small cross-shaped filter element is shown in Figures 7, 8 and 9. The filter element has a frame 70 which includes the inlet end 72, terminal plate 74 and ribs 76 and 78. The media 80 is over-molded to the frame 70 so as to form the cross-shape and pleats such as 82 and 84. Internal projections and grooves forming external shoulders are also provided. In Figure 8, the pleat-forming angle gamma (γ) is shown. Alpha (α) is about 0 degrees and beta (β) is about 90 degrees. Thus the pleat angle gamma (γ) is about equal to beta (β).

## Claims

1. A flow-through medical filter element which includes a filter medium (34) and frame-like means (32) for supporting and positioning said filter medium, wherein:

(a) said frame-like means (32) is a molded member and includes a ring-like inlet end (35), a fluid-impervious terminal end (36) spaced from said ring-like inlet, and a plurality of rib-like members (40, 42) which extend between and interconnect the inlet end and terminal end, said inlet and also having internally-extending pleat-supporting projections (48, 50) and said terminal end having complementary external pleat-supporting shoulders (52, 54, 56), said frame-like means (32) being symmetrical about an axial plane (43) and said external shoulders being formed at angles which are not re-entrant with respect to the said axial plane; and

(b) said filter medium having longitudinal pleats (44, 46) therein defining angles which are not re-entrant with respect to the said axial plane and said medium being bonded to the internal pleat-supporting projections (48, 50) at the inlet end and to the external pleat-supporting shoulders (52, 54, 56) at the terminal end.

2. A filter element as in Claim 1, whereby said terminal end shoulders (52, 54, 56) form a plurality of groove-like shapes in said terminal end which open outwardly from said axial plane.

3. A filter element as in Claim 2, wherein the angle (α) between the axial plane and a first groove-defining surface (53) is acute and the angle (β) between the axial plane and a second groove-defining surface (55) is obtuse.

4. A filter element as in any one of Claims 1 to 3, wherein the angle (α) between the axial plane and a first pleat surface is acute and the angle (β) between the axial plane and a second pleat surface is obtuse.

5. A filter element as in Claim 4, wherein the pleat angle (γ) is equal to the difference between (β) and (α).

6. A filter element as in Claim 4 or 5, wherein each said angle in the terminal end plate equals and is aligned with the corresponding angle in said pleats.

7. A filter element as in Claim 6, wherein said projections on the ring-like inlet end are aligned with the pleats of the filter medium.

8. A filter element as in Claim 7, wherein the filter element includes at least four pleats, with two pleats on each side of the parting line plane, and wherein at least one pleat-forming surface for each pleat does not lie along a plane passing through the longitudinal axis of the filter element.

9. A filter element as in Claim 8, wherein said element is symmetrical about a plane which includes the longitudinal axis of the element and which is transverse to the said axial plane (43).

10. A filter element as in any one of the preceding claims, wherein the filter medium is of the surface-type.

11. A filter element as in any one of the preceding claims, wherein one filter medium is of the depth-type.

12. A filter element as in any one of the preceding claims, wherein the filter medium is of unitary construction.

13. A filter element as in any one of the preceding claims, wherein the frame-like means (32) is molded to the filter medium (34).

**Patentansprüche**

1. Ein medizinisches Durchflußfilterelement mit einem Filtermedium (34) und einem rahmenartigen Element (32) zur Abstützung und Positionierung des Filtermediums, wobei:

(a) das rahmenartige Element (32) ein Formteil ist und ein ringförmiges Einlaßende (35), ein fluiddichtes, vom ringförmigen Einlaßende beabstandetes Abschlußende (36), und eine Vielzahl rippenartiger Teile (40, 42) aufweist, die sich unter Verbindung des Einlaßendes mit dem Abschlußende zwischen diesen erstrecken, wobei das Einlaßende auch sich inwendig erstreckende, Falten-unterstützende Vorsprünge (48, 50) aufweist, während das Abschlußende komplementäre, äußere Falten-unterstützende Schultern (52, 54, 56) umfaßt, wobei das rahmenartige Element (32), bezogen auf eine axiale Ebene (43), symmetrisch ist, und wobei die äußeren Schultern unter Winkeln gebildet sind, die bezüglichen der axialen Ebene nicht hinterschnitten sind; und wobei

(b) das Filtermedium langgestreckte Falten (44, 46) aufweist, die jeweils Winkel begrenzen, die bezüglich der genannten axialen Ebene nicht hinterschnitten sind, und wobei das genannte Medium mit den inneren, Falten-unterstützenden Vorsprüngen (48, 50) am Einlaßende und mit den äußeren, Falten-unterstützenden Schultern (52, 54, 56) am Abschlußende verklebt ist.

2. Filterelement nach Anspruch 1, wobei die genannten Schultern (52, 54, 56) am Abschlußende eine Vielzahl von nutartigen Umrissen in dem genannten Abschlußende bilden, die von der genannten axialen Ebene nach außen hin geöffnet sind.

3. Filterelement nach Anspruch 2, wobei der Winkel (α) zwischen der axialen Ebene und einer ersten Nut-begrenzenden Oberfläche (53) spitz ist, während der Winkel (β) zwischen der axialen Ebene und einer zweiten Nut-begrenzenden Oberfläche (55) stumpf ist.

4. Filterelement nach einem der Ansprüche 1 bis 3, wobei der Winkel (α) zwischen der axialen Ebene und einer ersten Falten-Oberfläche spitz ist, während der Winkel (β) zwischen der axialen Ebene und einer zweiten Falten-Oberfläche stumpf ist.

5. Filterelement nach Anspruch 4, wobei der Faltenwinkel (γ) gleich der Differenz zwischen (β) und (α) ist.

6. Filterelement nach Anspruch 4 oder 5, wobei jeder Winkel in der Abschlußendplatte mit dem entsprechenden Faltenwinkel gleich und fluchtend ist.

7. Filterelement nach Anspruch 6, wobei die genannten Vorsprünge am ringartigen Einlaßende mit den Falten des Filtermediums fluchten.

8. Filterelement nach Anspruch 7, wobei das Filterelement mindestens vier Falten aufweist, und zwar jeweils zwei Falten an jeder Seite der Trennlinienebene, und wobei mindestens eine Falten-unterstützende Oberfläche für jede Falte nicht in einer Ebene liegt, die sich durch die Längsachse des Filterelements erstreckt.

9. Filterelement nach Anspruch 8, wobei das genannte Element, bezogen auf eine Ebene, die die Längsachse des Elements umfaßt und die sich quer zur genannten axialen Ebene (43) erstreckt, symmetrisch ist.

10. Filterelement nach einem der vorangehenden Ansprüche, wobei das Filtermedium vom Oberflächentyp ist.

11. Filterelement nach einem der vorangehenden Ansprüche, wobei ein Filtermedium vom Tiefentyp ist.

12. Filterelement nach einem der vorhergehenden Ansprüche, wobei das Filtermedium von einheitlicher Konstruktion ist.

13. Filterelement nach einem der vorangehenden Ansprüche, wobei das rahmenartige Element (32) am Filtermedium (34) angeformt ist.

**Revendications**

1. Elément filtrant médical à écoulement traversant, qui comprend un milieu filtrant (34) et une ossature (32) pour supporter et positionner ledit milieu filtrant, dans lequel:

(a) ladite ossature (32) est une pièce moulée et elle comprend une extrémité d'entrée annulaire (35), une extrémité terminale imperméable au fluide (36) espacée de ladite entrée annulaire, et une pluralité de nervures (40, 42) qui s'étendent entre l'extrémité d'entrée et l'extrémité terminale et interconnectionnent celles-ci, ladite extrémité d'entrée comportant également des saillies de maintien de plis (48, 50) s'étendant vers l'intérieur, et ladite extrémité terminale comportant des épaulements complémentaires extérieurs de maintien de plis (52, 54, 56), ladite ossature (32) étant symétrique par rapport à un plan axial (43) et lesdits épaulements extérieurs étant formés suivant des angles qui ne sont pas rentrants par rapport audit plan axial, et

(b) ledit milieu filtrant comporte des plis longitudinaux (44, 46) définissant des angles qui ne sont pas rentrants par rapport audit plan axial, et ledit milieu filtrant est lié aux saillies intérieures de maintien de plis (48, 50) à l'extrémité d'entrée et aux épaulements extérieurs de maintien de plis (52, 54, 56) à l'extrémité terminale.

2. Elément filtrant suivant la revendication 1, dans lequel lesdits épaulements d'extrémité terminale (52, 54, 56) définissent une pluralité de configurations en forme de gorge dans ladite extrémité terminale, qui s'ouvrent vers l'extérieur par rapport audit plan axial.

3. Elément filtrant suivant la revendication 2, dans lequel l'angle (α) entre le plan axial et une première surface de définition de gorge (53) est aigu et l'angle (β) entre le plan axial et une deuxième surface de définition de gorge (55) est obtus.

4. Elément filtrant suivant l'une quelconque des revendications 1 à 3, dans lequel l'angle (α) entre le plan axial et une première surface de pli est aigu et l'angle (β) entre le plan axial et une deuxième surface de pli est obtus.

5. Elément filtrant suivant la revendication 4, dans lequel l'angle de pli (γ) est égal à la différence entre l'angle (β) et l'angle (α).

6. Elément filtrant suivant la revendication 4 ou 5, dans lequel chaque angle dans la plaque d'extrémité terminale est égal et aligné avec l'angle correspondant desdits plis.

7. Elément filtrant suivant la revendication 6 dans lequel lesdites saillies sur l'extrémité d'entrée annulaire sont alignées avec les plis sur le milieu filtrant.

8. Elément filtrant suivant la revendication 7, dans lequel ledit élément filtrant comprend au moins quatre plis, avec deux plis de chaque côté du plan de partage ou de joint, et dans lequel au moins une surface de formation de pli pour chaque pli ne se trouve pas dans un plan passant par l'axe longitudinal de l'élément filtrant.

9. Elément filtrant suivant la revendication 8, dans lequel ledit élément est symétrique par rapport à un plan qui contient l'axe longitudinal de l'élément et qui est transversal par rapport audit plan axial (43).

10. Elément filtrant suivant l'une quelconque des revendications précédentes, dans lequel le milieu filtrant est du type à filtrage en surface.

11. Elément filtrant suivant l'une quelconque des revendications précédentes, caractérisé en ce qu'un milieu filtrant est du type à filtrage en profondeur.

12. Elément filtrant suivant l'une quelconque des revendications précédentes, dans lequel le milieu filtrant est de construction unitaire.

13. Elément filtrant suivant l'une quelconque des revendications précédentes, dans lequel l'ossature (32) est liée par moulage avec le milieu filtrant (34).

FIG. 1

FIG. 2

FIG. 3

FIG. 4

1

FIG. 5

FIG. 6

FIG. 7

FIG. 8

FIG. 9

2